# EUROPÄISCHE PATENTSCHRIFT

(11) **EP 0 617 012 B1**
(45) Veröffentlichungstag und Bekanntmachung des Hinweises auf die Patenterteilung: **30.10.1996**
(21) Anmeldenummer: 94102863.1
(22) Anmeldetag: 25.02.1994
(51) Int. Cl.: C07C 251/54, C08F 8/30, C08J 3/24, C08L 33/06

(54) **Beta-Hydroxyoximether und bei Raumtemperatur mit beta-Hydroxyoximethern vernetzbare Dispersionen oder Lösungen**
Beta-hydroxy oxime ethers and dispersions or solutions which are cross-linkable at room temperature by beta-hydroxy oxime ethers
Bêta-hydroxy oxime éthers et dispersions ou solutions réticulables à température ambiante par des bêta-hydroxy oxime éthers

(30) Priorität: 02.03.1993 DE 4306392
(43) Veröffentlichungstag der Anmeldung: 28.09.1994
(73) Patentinhaber: BASF Aktiengesellschaft, 67063 Ludwigshafen (DE)
(72) Erfinder: Bauer, Gerhard, Dr., D-69469 Weinheim (DE); Aydin, Oral, Dr., D-68165 Mannheim (DE); Strecker, Beate, Dr., D-67059 Ludwigshafen (DE); Oftring, Alfred, Dr., D-67098 Bad Duerkheim (DE)

(56) Entgegenhaltungen:
- EP-A- 0 369 944
- EP-A- 0 516 074
- DE-A- 2 658 938

## Beschreibung

Die Erfindung betrifft eine Verbindung der Formel 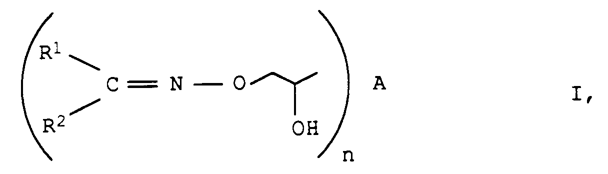 worin R¹ und R² unabhängig voneinander für einen C₁-C₁₀ Alkyl, C₁-C₁₀ Alkoxy-, C₅-C₁₀-Cycloalkyl- oder einen C₅-C₁₀ Arylrest, welche auch 1 bis 3 nicht benachbarte Stickstoff-, Sauerstoff- oder Schwefelatome als Heteroatome in der Kohlenstoffkette oder Kohlenstoffring enthalten und durch 1 bis 3 C₁-C₄-Alkyl- oder -Alkoxygruppen substituiert sein können, stehen, R¹ oder R² für ein Wasserstoffatome stehen können, oder R¹ und R² gemeinsam eine Brücke aus 2 bis 14 Kohlenstoffatomen bilden, wobei ein Teil der Kohlenstoffatome auch Bestandteil eines aromatischen Ringsystems sein kann, A für einen n-wertigen organischen Rest steht und n eine ganze Zahl gleich oder größer 2 ist.

Desweiteren betrifft die Erfindung Dispersionen oder Lösungen von radikalischen Polymerisaten, Polykondensaten oder Polyaddukten, welche Verbindungen der Formel I enthalten.

Bei Copolymerisaten, welche in Beschichtungsmitteln oder Klebstoffen Verwendung finden, handelt es sich vielfach um vernetzungsfähige Copolymerisate. Durch eine Vernetzung können z.B. Schutzüberzüge, Lackierungen oder Klebstoffbeschichtungen mit guten elastischen Eigenschaften, hoher Kohäsion, d.h. innere Festigkeit, hoher Chemikalien- und Lösemittelbeständigkeit erhalten werden.

Zur Vernetzung wird den Copolymerisaten im allgemeinen ein Vernetzungsmittel zugesetzt, das mit funktionellen Gruppen im Copolymerisat reagiert.

Mögliche Vernetzungsmittel sind z.B. Polyisocyanate, welche mit Hydroxyl- oder Aminogruppen reagieren.

Aus der DE-A-35 21 618 sind entsprechende wäßrige Klebstoffzubereitungen bekannt, bei denen in Wasser dispergierte Polyisocyanate wäßrigen Dispersionen radikalische polymerisierter Copolymerisate als Vernetzungsmittel zugesetzt werden. Ähnliche Klebstoffzubereitungen sind auch in der US-A 4 396 738 und DE-A-31 12 117 beschrieben.

Nachteilig bei diesen wäßrigen Zubereitungen ist jedoch die mangelnde Lagerstabilität. Das Polyisocyanat darf daher erst kurz vor seiner Verwendung als Vernetzungshilfsmittel in Wasser dispergiert und mit dem Copolymerisat gemischt werden.

Eine erhöhte Lagerstabilität kann durch Umsetzung der Isocyanatgruppen mit Blockierungsmitteln, z.B. Oximen, Caprolactam, Phenolen, Maleinsäuredialkylestern erreicht werden. Die erhaltenen sog. blockierten Polyisocyanate hydrolysieren in wäßriger Dispersion nur noch in untergeordnetem Ausmaß.

Gegenstand der DE-A-38 07 555 ist ein solches, mit Oximen blockiertes Diisocyanat, welches in Wasser dispergiert wird und sich als Zusatz für in Wasser dispergierte Polymerisate eignet.

Vernetzungsreaktionen treten jedoch erst nach Abspaltung des Blockierungsmittels bei Temperaturen ab ca. 130°C auf.

Bisher bekannte wäßrige Klebstoffzubereitungen mit Polyisocyanaten als Vernetzungshilfsmittel sind daher entweder nicht lagerstabil und können daher nur als 2-Komponentensystem Verwendung finden oder vernetzen erst bei hohen Temperaturen.

Lagerstabile, bei Raumtemperatur nach Entfernen des Lösungsmittels vernetzende wäßrige Dispersionen sind aus der EP-A-3516 bekannt. Diese Dispersionen enthalten Polyhydrazide, welche mit im Copolymerisat einpolymerisierten Monomeren mit Carbonylgruppen reagieren.

Aus der nicht vorveröffentlichten deutschen Patentanmeldung P 42 19 384.2 sind Oximether als Vernetzungsmittel bekannt. In der EP-A-516 074 sind Aminooxyderivate als Vernetzer für Keto- oder Aldehydgruppen enthaltende Copolymerisate beschrieben.

Grundsätzlich besteht ein Bedarf an weiteren, bei Raumtemperatur vernetzenden Dispersionen, um Alternativen zur Polyhydrazidvernetzung zu Verfügung stellen zu können. Des weiteren sollen diese Dispersionen gute anwendungstechnische Eigenschaften, z.B. eine gute Haftung, insbesondere Naßhaftung auf unterschiedlichsten Substraten, aufweisen.

Aufgabe der vorliegenden Erfindung waren daher lagerstabile Dispersionen oder Lösungen von vernetzbaren Copolymerisaten, welche ein Vernetzungsmittel enthalten und bei Raumtemperatur vernetzbar sind.

Demgemäß wurde die oben definierte Verbindung, sowie Dispersionen oder Lösungen, welche diese Verbindung enthalten, gefunden.

Die Verbindung der Formel I eignet sich als Vernetzungsmittel oder Haftungsvermittler in Dispersionen oder Lösungen von radikalischen Polymerisaten, Polykondensaten oder Polyaddukten.

Die Vernetzung von Keto- oder Aldehydgruppen enthaltenden radikalischen Polymerisate, Polykondensate oder Polyaddukte mit Vernetzungsmitteln der Formel I tritt bei Entfernung der flüssigen Phase der Dispersion oder Lösung ein.

Vernetzende Gruppen sind die β-Hydroxyoximethergruppen 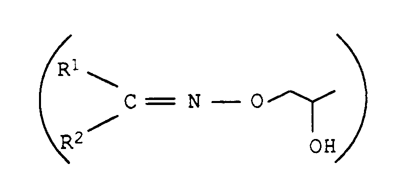 welche z.B. mit Keto- oder Aldehydgruppen reagieren.

Bevorzugt enthält die Dispersion oder Lösung, welcher Verbindungen I als Vernetzungmittel zugesetzt werden, daher ein Polymerisat, Polykondensat oder Polyaddukt, das zu 0,001 bis 20 Gew.-%, bevorzugt 0,01 bis 10 Gew.-% und ganz besonders bevorzugt zu 0,05 bis 3 Gew.-% aus Aldehydgruppen -CHO- oder Ketogruppen -CO- besteht.

Es kann sich dabei z.B. um ein radikalisch polymerisiertes Copolymerisat, ein Polyester als Polykondensat oder ein Polyurethan als Polyaddukt handeln.

Im Falle der radikalisch polymerisierten Copolymerisate sind die Aldehyd- oder Ketogruppen vorzugsweise durch ethylenisch ungesättigte Verbindungen, welche diese Gruppen enthalten, einpolymerisiert.

Vorzugsweise handelt es sich um ethylenisch ungesättigte Verbindungen mit ein oder zwei Aldehyd-, bzw. Ketogruppen oder einer Aldehyd- und einer Ketogruppe und einer radikalisch polymerisierbaren olefinischen Doppelbindung (im weiteren Monomere a) genannt).

Im Falle eines Polyesters kann es sich z.B. um Monoalkohole, Diole, Monocarbonsäuren oder Dicarbonsäuren, im Falle eines Polyurethans kann es sich z.B. um Mono-, Diisocyanate oder ebenfalls Monoalkohole oder Diole handeln, welche Aldehyd- oder Ketogruppen enthalten.

Als Monoalkohole genannt seien z.B. Hydroxyaceton, Hydroxybenzaldehyd, Acetoin und Benzoin.

Geeignete Monocarbonsäuren sind z.B. Ketocarbonsäuren, wie Brenztraubensäure oder Lävulinsäure.

Ferner können Verbindungen mit Aldehyd- oder Ketogruppen in die Polymerisate, Polykondensate oder Polyaddukte nicht nur als Bestandteil der Hauptkette, sondern auch durch Umsetzung mit reaktiven Gruppen in der Polymerhauptkette an die Polymerisate, Polykondensate oder Polyaddukte gebunden werden.

Bevorzugt ist ein radikalisch polymerisiertes Copolymerisat, welches aus den Monomeren a), welche Aldehyd- oder Ketogruppen enthalten, und weiteren Monomeren b) und c) besteht.

Als Monomere a) kommen z.B. Acrolein, Methacrolein, Vinylalkylketone mit 1 bis 20, vorzugsweise 1 bis 10 Kohlenstoffatomen im Alkylrest, Formylstyrol, (Meth-)acrylsäurealkylester mit ein oder zwei Keto- oder Aldehyd-, bzw. einer Aldehyd- und einer Ketogruppe im Alkylrest, wobei der Alkylrest vorzugsweise insgesamt 3 bis 10 Kohlenstoffatome umfaßt, z.B. (Meth)acryloxyalkylpropanale, wie sie in der DE-A-27 22 097 beschrieben sind. Des weiteren eignen sich auch N-Oxoalkyl(meth)acrylamide wie sie z.B. aus der US-A-4 226 007, der DE-A-20 61 213 oder DE-A-22 07 209 bekannt sind.

Besonders bevorzugt sind Acetoacetyl(meth)acrylat, Acetoacetoxyethyl(meth)acrylat und insbesondere Diacetonacrylamid.

Die Hauptmonomeren b) sind in dem Copolymerisat insbesondere zu 20 bis 99,99, bevorzugt 60 bis 99,9 und besonders bevorzugt zu 80 bis 99,5 Gew.-%, bezogen auf das Copolymerisat, enthalten.

Als Monomere b) kommen Ester der Acryl- oder Methacrylsäure von 1 bis 20 C-Atome enthaltenden Alkylalkoholen in Frage. Als solche Alkohole seien genannt Methanol, Ethanol, n- oder i-Propanol, n-, s- und t-Butanol, n-Pentanol, Isoamylalkohol, n-Hexanol, Octanol, 2-Ethylhexanol, Lauryl- und Stearylalkohol.

Gute Ergebnisse werden mit (Meth)-acrylsäurealkylestern mit einem C₁-C₁₀-Alkylrest, wie Methylmethacrylat, Methylacrylat, n-Butylacrylat, Ethylacrylat und 2-Ethylhexylacrylat erzielt.

Insbesondere sind auch Mischungen der (Meth)acrylsäurealkylester geeignet.

Weiterhin kommen Vinylester von Carbonsäuren mit 1 bis 20 C-Atomen wie Vinyllaurat, -stearat, Vinylpropionat und Vinylacetat in Betracht.

Als vinylaromatische Verbindungen mit bis zu 20 C-Atomen kommen Vinyltoluol, a- und p-Styrol, α-Butylstyrol, 4-n-Butylstyrol, 4-n-Decylstyrol und vorzugsweise Styrol in Betracht. Beispiele für ethylenisch ungesättigte Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als nicht aromatische Kohlenwasserstoffe mit 2 bis 8 C-Atomen und mindestens zwei konjugierten olefinischen Doppelbindungen seien Butadien, Isopren und Chloropren genannt.

Die Monomeren b) können insbesondere auch im Gemisch eingesetzt werden, vor allem, um gewünschte Glasübergangstemperaturen des Copolymerisats einzustellen.

Als weitere, d.h. nicht unter a) und b) fallende, copolymerisierbare Monomere c) kommen z.B. Ester der Acryl- und Methacrylsäure von Alkoholen mit 1 bis 20 C-Atomen, die außer dem Sauerstoffatom in der Alkoholgruppe mindestens ein weiteres Heteroatom enthalten und/oder die einen aliphatischen oder aromatischen Ring enthalten, in Betracht.

Genannt seien z.B. 2-Ethoxyethylacrylat, 2-Butoxyethyl(meth)acrylat, Dimethylaminoethyl(meth)acrylat, Diethylaminoethyl(meth)acrylat, (Meth)acrylsäurearyl-, -alkaryl- oder cycloalkylester, wie Cyclohexyl(meth)acrylat, Phenylethyl(meth)-acrylat, Phenylpropyl(meth)acrylat- oder Acrylsäureester von heterocyclischen Alkoholen wie Furfuryl- (meth)acrylat.

Darüber hinaus seien noch weitere Comonomere wie (Meth)acrylamid sowie deren am Stickstoff mit C₁-C₄-Alkyl substituierten Derivate genannt.

Von besonderer Bedeutung sind auch hydroxyfunktionelle Comonomere, z.B. (Meth)acrylsäure-C₁-C₁₅-alkylester, welche durch ein oder zwei Hydroxygruppen substituiert sind. Insbesondere von Bedeutung als hydroxyfunktionelle Comonomere sind (Meth)acrylsäure-C₁-C₈-Hydroxyalkylester, wie n-Hydroxyethyl-, n-Hydroxypropyl- oder n-Hydroxybutyl(meth)acrylat.

Die Mitverwendung von Comonomeren mit salzbildenden Gruppen empfiehlt sich z.B. für die Herstellung selbstdispergierbarer Copolymerisate, die z.B. für wäßrige Sekundärdispersionen geeignet sind. Comonomere mit salzbildenden Gruppen sind insbesondere Itaconsäure, Acrylsäure und Methacrylsäure.

Der Gewichtsanteil der weiteren Comonomeren im Copolymerisat kann insbesondere 0 bis 50 %, vorzugsweise 0 bis 20 % und ganz besonders bevorzugt 0 bis 10 Gew.% betragen.

Die Anteile der Monomeren a), b) und c) addieren sich zu 100 Gew.-%.

Der Anteil der Monomeren a) wird dabei so gewählt, daß der oben angegebene Gehalt an Aldehyd- oder Ketogruppen im Copolymerisat vorliegt.

Die Herstellung des Copolymerisats erfolgt im allgemeinen durch radikalische Polymerisation. Geeignete Polymerisationsmethoden, wie Substanz-, Lösungs-, Suspensions- oder Emulsionspolymerisation sind dem Fachmann bekannt.

Vorzugsweise wird das Copolymerisat durch Lösungspolymerisation mit anschließender Dispergierung in Wasser oder besonders bevorzugt durch Emulsionspolymerisation hergestellt, wobei das Copolymerisat als wäßrige Dispersion erhalten wird.

Die Comonomeren können bei der Emulsionspolymerisation wie üblich in Gegenwart eines wasserlöslichen Initiators und eines Emulgators bei vorzugsweise 30 bis 95°C polymerisiert werden.

Geeignete Initiatoren sind z.B. Natrium-, Kalium- und Ammoniumpersulfat, tert.-Butylhydroperoxide, wasserlösliche Azoverbindungen oder auch Redoxinitiatoren.

Als Emulgatoren dienen z.B. Alkalisalze von längerkettigen Fettsäuren, Alkylsulfate, Alkylsulfonate, alkylierte Arylsulfonate oder alkylierte Biphenylethersulfonate.

Des weiteren kommen als Emulgatoren Umsetzungsprodukte von Alkylenoxiden, insbesondere Ethylen- oder Propylenoxid mit Fettalkoholen, -säuren oder Phenol, bzw. Alkylphenolen in Betracht.

Im Falle von wäßrigen Sekundärdispersionen wird das Copolymerisat zunächst durch Lösungspolymerisation in einem organischen Lösungsmittel hergestellt und anschließend unter Zugabe von Salzbildnern, z.B. von Ammoniak zu Carbonsäuregruppen enthaltenden Copolymerisaten, in Wasser ohne Verwendung eines Emulgators oder Dispergierhilfsmittels dispergiert. Das organische Lösungsmittel kann abdestilliert werden. Die Herstellung von wäßrigen Sekundärdispersionen ist dem Fachmann bekannt und z.B. in der DE-A-37 20 860 beschrieben.

Zur Einstellung des Molekulargewichts können bei der Polymerisation Regler eingesetzt werden. Geeignet sind z.B. -SH enthaltende Verbindungen wie Mercaptoethanol, Mercaptopropanol, Thiophenol, Thioglycerin, Thioglykolsäureethylester, Thioglykolsäuremethylester und tert.-Dodecylmercaptan.

Die Art und Menge der Comonomeren wird zweckdienlicherweise so gewählt, daß das erhaltene Copolymerisat eine Glasübergangstemperatur zwischen bevorzugt -60 und +140°C. Je nachdem, ob harte oder weiche Beschichtungen gewünscht sind, werden durch Wahl der Monomeren hohe oder tiefe Glasübergangstemperaturen eingestellt. Die Glasübergangstemperatur des Copolymerisats läßt sich nach üblichen Methoden wie Differentialthermoanalyse oder Differential Scanning Calorimetrie (s. z.B. ASTM 3418/82, sog. "midpoint temperature") bestimmen.

Die Dispersion oder Lösung enthält als Haftungsvermittler oder als Vernetzungsmittel eine Verbindung der Formel I.

In der Formel I können R¹ und R² unabhängig voneinander für einen C₁-C₁₀-Alkyl, einen C₁-C₁₀-Alkoxy-, einen C₅-C₁₀-Cycloalkyl- oder einen C₅-C₁₀-Arylrest, welche auch 1 bis 3 nicht benachbarte Stickstoff-, Sauerstoff- oder Schwefelatome in der Kohlenstoffkette oder im Kohlenstoffring enthalten und durch 1 bis 3 C₁-C₄-Alkyl- oder -Alkoxygruppen substituiert sein können, stehen, weiterhin können R¹ oder R² zusätzlich die Bedeutung eines Wasserstoffatoms haben,
oder können R¹ und R² gemeinsam eine Brücke aus 2 bis 14 Kohlenstoffatomen bilden, wobei ein Teil der Kohlenstoffatome auch Bestandteil eines aromatischen Ringsystems sein kann.

Die Reste R¹ und R² stehen jeweils bevorzugt für ein Wasserstoffatom, eine C₁- bis C₆-Alkylgruppe oder eine C₁- bis C₆-Alkoxygruppe. Im Falle des Wasserstoffatoms kann nur einer der Reste R¹ oder R² für ein Wasserstoffatom stehen.

Als organischer Rest A in Betracht kommen z.B. ungesättigte oder vorzugsweise gesättigte lineare, verzweigte oder cyclische Kohlenwasserstoffreste, welche gegebenenfalls durch nicht benachbarte Stickstoff-, Schwefel-, insbesondere Sauerstoffatome unterbrochen sein können. Besonders bevorzugt genannt seien C₂- bis C₈-Alkylengruppen oder Gruppen der Formel -O- (CH₂)ₓ-O- mit X = 2 bis 20.

Desweiteren kommen als Reste A auch aromatische Kohlenwasserstoffreste oder Kohlenwasserstoffreste in Betracht, welche sowohl aliphatische als auch aromatische Kohlenwasserstoffgruppen enthalten.

Als letztere genannt seien insbesondere C₅-C₁₂-Arylenreste oder Reste der Formel 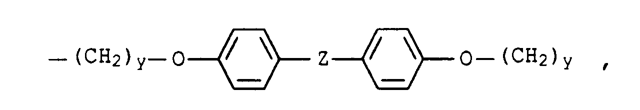
worin Z für einen linearen oder verzweigten aliphatischen Kohlenwasserstoffrest mit 1 bis 5 Kohlenstoffatomen und y für eine ganze Zahl von 1 bis 3 steht.

Der Rest A kann aber z.B. auch ein polymerer Rest sein, wie er z.B. durch Polykondensation von Bisphenol A mit Epichlorhydrin erhältlich ist.

Die Variable n steht für eine ganze Zahl gleich oder größer 2. Vorzugsweise hat n einen Wert von 2 bis 30, besonders bevorzugt von 2 bis 4. Ganz besonders bevorzugt ist n = 2.

Für die Verwendung als Vernetzer oder Haftungsvermittler sind generell niedermolekulare Verbindungen ausreichend und der Einsatz von höhermolekularen Verbindungen daher nicht notwendig. Das Molekulargewicht der Verbindungen I liegt daher im allgemeinen unter 5000 vorzugsweise unter 1 000 g/Mol.

Vorzugsweise beträgt der Gewichtsanteil der Verbindung der Formel I in den Dispersionen der Lösungen 0,01 bis 30 Gew.-%, besonders bevorzugt 0,1 bis 20 Gew.-%, ganz besonders bevorzugt 0,1 bis 5 Gew.-%, bezogen auf das Polymerisat, Polykondensat oder Polyaddukt. Vorteilhafterweise wird der Gehalt im Falle Keto- oder Aldehydgruppen enthaltender radikalischer Polymerisate, Polykondensate oder Polyaddukte so gewählt, daß die β-Hydroxyoximethergruppen im ungefähr äquimolaren Verhältnis zu den Keto- und/oder Aldehydgruppen vorliegen.

In den Dispersionen oder Lösungen können die Verbindungen z.B. in Gegenwart von Säuren gegebenenfalls teilweise hydrolysieren, so daß auch in geringen Mengen β-Hydroxyaminooxyverbindungen bzw. -gruppen entstehen. Diese β-Hydroxyaminooxygruppen können mit Keto- oder Aldehydgruppen ebenso vernetzen und tragen auch zu einer Haftungsverbesserung bei.

Metallsalze oder Metallkomplexe, welche in den Dispersionen oder Lösungen vorhanden sind bzw. zugesetzt werden, beeinträchtigen die Vernetzung oder Haftungsverbesserung durch Verbindungen I im allgemeinen nicht.

Die Verbindungen der Formel I können in einfacher Weise hergestellt werden durch Umsetzung von Epoxyverbindungen mit Oximen. Als Epoxyverbindungen kommen z.B. Alkandioldiglycidether der Formel 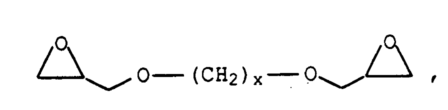 epoxidierte α-Olefine der Formel 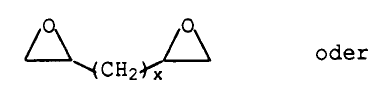 Bisphenoldiglycidether der Formel 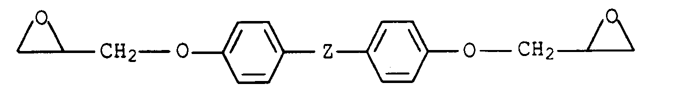 insbesondere der Bisphenol-A-diglycidether in Betracht, worin Z die oben angegebene Bedeutung hat.

Als Oxime eignen sich z.B. solche von aliphatischen, cycloaliphatischen oder aromatischen Aldehyden oder Ketonen, z.B. Acetonoxim, Methylethylketonoxim, Dieethylketoxim, Methylisopropylketoxim, Methylisobutylketoxim, Diisopropylketoxim, Cyclohexanonoxim, 2-Methylcyclohexanonoxim, 2,6-Dimethylcyclohexanonoxim, Acetophenonoxim, Benzophenonoxim oder Diethylglyoxim, geeignet. Bevorzugt sind Oxime von aliphatischen Ketonen oder Aldehyden mit einer Ketogruppe oder Aldehydgruppe und insgesamt 3 bis 12 Kohlenstoffatomen, insbesondere Acetonoxim und Methylethylketonoxim.

Die Herstellung der Verbindungen I kann in einfacher Weise dadurch erfolgen, daß das Oxim in einem ersten Schritt mit einer Base, z.B. NaOH oder einem sonstigen Nucleophil, umgesetzt wird. Dabei entsteht voraussichtlich ein Anion mit einer negativen Ladung am zum Oxim-Stickstoff benachbarten Sauerstoffatom. Das Reaktionsprodukt kann dann mit der Epoxidverbindung z.B. bei 0 bis 100°C insbesondere bei 50 bis 80°C zur Verbindung I umgesetzt werden.

Der Feststoffgehalt der erfindungsgemäßen Dispersion oder Lösung liegt bevorzugt zwischen 20 und 90 Gew.-%, insbesondere zwischen 30 und 70 Gew.-%.

Die erfindungsgemäßen Dispersionen oder Lösungen eignen sich als Beschichtungsmittel für unterschiedliche Substrate mit Kunststoff-, Holz- oder Metalloberflächen oder z.B. für Textilien, Fliesstoffe, Leder oder Papier. Sie eignen sich ebenfalls für Anwendungen in der Bauchemie z.B. als Klebstoffe, Dichtungsmassen, Bindemittel oder ähnliches. Bei den Beschichtungen kann es sich z.B. um Anstriche, Schutzüberzüge, Lackierungen mit Schutzfunktionen, pigmentierte oder unpigmentierte Lackierungen, auch für dekorative Zwecke, oder Klebstoffbeschichtungen handeln.

Insbesondere ist für die genannten Verwendungen eine wäßrige Dispersion eines radikalisch polymerisierten, insbesondere eines Keto- und/oder Aldehydgruppen enthaltenden Copolymerisats geeignet.

Die wäßrige Dispersion kann auch organische, vorzugsweise mit Wasser mischbare Lösungsmittel als Hilfslösungsmittel enthalten.

Die erfindungsgemäße Dispersion oder Lösung kann je nach Verwendungszweck übliche Hilfs- und Zusatzmittel enthalten. Hierzu gehören beispielsweise Füllstoffe wie Quarzmehl, Quarzsand, hochdisperse Kieselsäure, Schwerspat, Calciumcarbonat, Kreide, Dolomit oder Talkum, die oft zusammen mit geeigneten Netzmitteln wie z.B. Polyphosphaten wie Natriumhexamethaphosphat, Naphthalinsulfonsäure, Ammonium- oder Natriumpolyacrylsäuresalze eingesetzt werden, wobei die Netzmittel im allgemeinen von 0,2 bis 0,6 Gew.-%, bezogen auf den Füllstoff, zugesetzt werden.

Bei der Verwendung für Lackierungen kann die erfindungsgemäße Dispersion oder Lösung lacktypische Zusätze wie Filmbildehilfsmittel, Pigmente, Mattierungsmittel, Verdicker, Pigmentdispergiermittel, Entschäumer etc. und auch andere natürlich oder synthetische Harze, z.B. Alkydharze, Polyurethane etc., enthalten.

Fungizide zur Konservierung werden, falls gewünscht im allgemeinen in Mengen von 0,02 bis 1 Gew.-%, bezogen auf die gesamte Dispersionen oder Lösung eingesetzt. Geeignete Fungizide sind beispielsweise Phenol- oder Kresol-Derivate oder zinnorganische Verbindungen.

Besonders eignet sich die erfindungsgemäßen Dispersionen oder Lösungen, insbesondere als wäßrige Dispersion eines radikalischen Copolymerisats, als Dichtstoff oder Klebstoff, insbesondere z.B. als Kaschierklebstoff zur Herstellung von Verbundfolien und Glanzfolien. Als Klebstoff können die Dispersionen neben obengenannten Zusatzstoffen noch spezielle, in der Klebstofftechnologie übliche Hilfs- und Zusatzmittel enthalten. Hierzu gehören beispielsweise Verdickungsmittel, Weichmacher oder auch klebrigmachende Harze wie z.B. Naturharze oder modifizierte Harze wie Kolophoniumester oder synthetische Harze wie Phthalatharze.

Dispersionen, welche als Klebstoff Verwendung finden, enthalten besonders bevorzugt Alkyl(meth)acrylate als Comonomere b) im Copolymerisat.

Die Glasübergangstemperatur der Copolymerisate wird bei der Verwendung als Klebstoffzubereitung bevorzugt auf Werte zwischen 0 und -40°C eingestellt.

Die Dispersionen zeigen bei der Verwendung als Klebstoff überraschend auch eine sehr gute Haftung, insbesondere Naßhaftung.

Der pH-Wert der Dispersion wird bevorzugt auf einen Wert zwischen 2 und 9 eingestellt, da die Vernetzungsreaktion mit den Copolymerisaten sauer katalysiert werden kann.

Die erfindungsgemäße Dispersionen oder Lösungen, welche Verbindungen der Formel I enthalten, sind lagerstabil. Die Vernetzungsreaktion, z.B. mit Keto- und/oder Aldehydgruppen, tritt schon bei Raumtemperatur bei Entfernen der flüssigen Phase, z.B. Verflüchtigung des Wassers ein.

Durch Temperaturerhöhung z.B. auf 30 bis 100°C kann die Verflüchtigung des Wassers beschleunigt werden.

Bei der Beschichtung von Substraten ist es prinzipiell auch möglich, eine Dispersion oder Lösung des Polymerisats, Polykondensats oder Polyaddukts, welche die Hydroxylamine oder Oximetherderivate nicht enthält, auf eine Oberfläche aufzutragen, auf die bereits vorher in einem getrennten Arbeitsgang Verbindungen der Formel I aufgebracht wurden.

In diesem Fall wirken die Verbindungen als sog. Primer.

Nach Auftragen der Dispersion oder Lösung tritt dann die Vernetzung ein oder zeigt sich eine Haftungsverbesserung.

### Beispiele

### Herstellung der Copolymerisate

### Copolymerisatdispersion 1

In einem Reaktionsgefäß mit Rührer und zwei Zulaufgefäßen (Zulauf 1 und Zulauf 2) wurden 200 g entsalztes Wasser, 37 g des Zulaufs 1 (siehe unten) und 20 g des Zulaufs 2 vorgelegt und auf 85°C erwärmt. Nach 15 Minuten wurde innerhalb von 2 h gleichmäßig der Zulauf 1 sowie innerhalb von 2,5 h gleichmäßig der Zulauf 2 zum Reaktionsgefäß zugegeben. Nach der letzten Initiator-Zugabe (Zulauf 2) wurde die Dispersion noch 1 Stunde bei 85°C gerührt.

### Zulauf 1: (dieser Zulauf wird während der Polymerisation gerührt)

- 107,5 g: entsalztes Wasser
- 400 g: Ethylacrylat
- 90 g: Methylmethacrylat
- 50 g: 20 gew.-%ige wäßrige Diacetonacrylamid-Lösung
- 50 g: 20 gew.-%ige Lösung des Natriumsalzes vom p-Dodecyldiphenyletherdisulfonat in Wasser (Emulgator)
- 50 g: 20 gew.-%ige Lösung des Umsetzungsprodukts von p-Isononylphenol mit ca. 50 Mol Ethylenoxid in Wasser (Emulgator)

### Zulauf 2:

- 100 g: entsalztes Wasser
- 3 g: Natriumpersulfat

Copolymerisatdispersionen 2 und 3 wurden entsprechend hergestellt (Tabelle 1).

**Tabelle 1**

| Zusammensetzung der Copolymerisate in Gew.-% | | | | |
|---|---|---|---|---|
| Copolymerisatdispersion | EA | MMA | DAA | AAEM |
| 1 | 80 | 18 | 2 | |
| 2 | 96 | | 4 | |
| 3 | 77,7 | 17,4 | | 4,9 |
| Abkürzungen EA: Ethylacrylat MMA: Methylmethacrylat DAA: Diacetonacrylamid AAEM: Acetoacetoxyethylmethacrylat | | | | |

### Herstellung der Vernetzer

### Vernetzer A

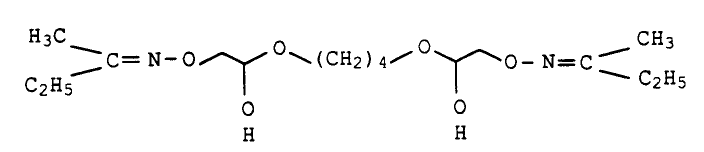

100 g (1,15 Mol) Butanonoxim wurden in 100 ml Toluol vorgelegt. Bei Raumtemperatur wurden dann 40 g (0,50 Mol) 50 gew.%ige NaOH in ca. 20 Minuten zugetropft. Die Temperatur stieg dabei auf ca. 35°C. Das Reaktionsgemisch wurde bei 120°C unter Rückfluß erhitzt und insgesamt 24,3 g Wasser abdestilliert.

Die erhaltene Reaktionslösung wurde unter Stickstoff auf 50°C abgekühlt und innerhalb von 6 Stunden 225,1 g (0,57 Mol) Butandioldiglycidether zugetropft. Die Mischung wurde noch eine Stunde bei einer Temperatur von 50°C gehalten, danach war kein unumgesetztes Oxid mehr nachweisbar. Die organische Lösung wurde zweimal mit Wasser extrahiert. Aus den vereinigten wäßrigen Phasen wurde restliches Toluol abdestilliert. Es wurde schließlich 336 g einer orangefarbenen wäßrigen Lösung des Vernetzers A mit einem Feststoffgehalt von 24,1 Gew.-% erhalten.

### Vernetzer B

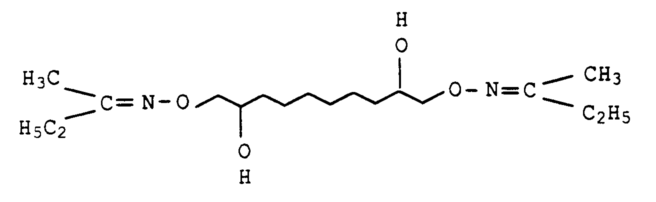

Es wurde verfahren wie bei Vernetzer A beschrieben. Statt Butandioldiglycidether wurde jedoch α, ω Diepoxydecan zugesetzt. Nachdem kein unumgesetztes Oxid mehr nachweisbar war, wurde das Toluol bei 70 bis 80°C im Vakuum (< 10 mbar) entfernt. Der Vernetzer B fiel als rotbraune, viskose Flüssigkeit an.

Prüfung auf Vernetzbarkeit (Quellung) und anwendungstechnische Prüfung (Glanzfolienprüfung und Verbundfolienprüfung)
Für die Prüfungen wurden die Vernetzer A bzw. B den Dispersionen 1 bis 3 jeweils in äquimolaren Mengen, bezogen auf die Keto- bzw. Aldehydgruppen zugesetzt.

### Quellung

Die Dispersionszubereitungen wurden verfilmt und die Filme 1 Woche bei Raumtemperatur getrocknet. Anschließend wurde das Quellungsverhalten als Maß für den Vernetzungsgrad dieser Filme in Tetrahydrofuran untersucht, indem ca. 1 g der verfilmten Proben in Tetrahydrofuran 1 Tag gelagert und die Lösungsmittelaufnahme in % gemessen wurde.

Mit zunehmender Vernetzungsdichte kommt es zu einer Abnahme der Lösungsmittelaufnahme bei der Quellung.

Nicht oder kaum vernetzte Polymere werden aufgelöst oder quellen aufgrund der außerordentlich geringen Vernetzungsdichte übermäßig stark an.

### (Ergebnisse in Tab. 2).

### Glanzfolienprüfung

Die Dispersionszubereitungen wurden mit einer Trockenschichtdicke von 5 g/m² auf mit Offsetfarben bedruckten Kartonagen gerakelt, bei 60°C getrocknet und nach ca. 30 sec mit biaxial verstreckten Polypropylenfolien (o-PP) kaschiert.

Es wurde geprüft, ob es beim Abreißen der Folie (Schälprüfung) zu einem Papier- und Farbausriß von der Kartonage kommt (Note 1: vollständiger Papier- oder Farbausriß, Note 2: teilweiser Papier- oder Farbausriß) und ob sich im Bereich von Nuten (Prägungen in der Kartonage) die Folie ablöst bzw. nicht fest anliegt (Nutstandfestigkeit; + = keine Ablösung an der Nut, - = Ablösung an der Nut und +/- = teilweise Ablösung an der Nut).

### (Ergebnisse in Tab. 2).

### Verbundfolienprüfung

Die Dispersionszubereitungen wurden mit einer Trockenschichtdicke von 3 g/m² auf eine auf 50°C erwärmte Polyethylentherephthalatfolie (PETP) gerakelt und nach 20 sek. mit einer Polyethylenfolie PE (coronavorbehandelt) kaschiert. Anschließend wurden die Folien 7 Tage bei Raumtemperatur und Normalklima gelagert und danach in 2 cm breite Streifen zerschnitten. Diese Streifen wurden dann bei 23°C im Winkel von 180°C mit einer Geschwindigkeit von 100 m/min abgezogen. Es wurde die Schälkraft in N bei den 2 cm breiten Streifen bestimmt (Tabelle 2).

**Tabelle 2**

| Prüfergebnisse | | | | | |
|---|---|---|---|---|---|
| Dispersion | Vernetzer | Quellung | Glanzfolienkaschierung | | Verbundfolienkaschierung |
| | | Lösungsmittelaufnahme Gew.-% | Schälprüfung | Nutstandfestigkeit | Schälfestigkeit |
| 1 | - | -* | 2 | - | 0,5 |
| 2 | - | -* | 2 | - | 0,5 |
| 3 | - | -* | 2 | - | 0,5 |
| 1 | A | 1940 | 1 | + | 1,9 |
| 2 | A | 1800 | 1 | + | 2,2 |
| 3 | A | 2000 | 1 | + | 2,0 |
| 1 | B | 2100 | 1 | + | 1,5 |
| 2 | B | 1920 | 1 | + | 1,9 |
| 3 | B | 2060 | 1 | + | 1,6 |

| | | | | | |
|---|---|---|---|---|---|
| * Film wird aufgelöst | | | | | |

## Patentansprüche

1. Verbindung der Formel 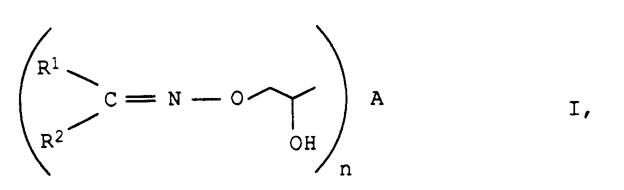 worin R¹ und R² unabhängig voneinander für einen C₁-C₁₀ Alkyl, C₁-C₁₀ Alkoxy-, C₅-C₁₀-Cycloalkyl- oder einen C₅-C₁₀ Arylrest, welche auch 1 bis 3 nicht benachbarte Stickstoff-, Sauerstoff- oder Schwefelatome als Heteroatome in der Kohlenstoffkette oder Kohlenstoffring enthalten und durch 1 bis 3 C₁-C₄-Alkyl- oder -Alkoxygruppen substituiert sein können, stehen, R¹ oder R² für ein Wasserstoffatome stehen können, oder R¹ und R² gemeinsam eine Brücke aus 2 bis 14 Kohlenstoffatomen bilden, wobei ein Teil der Kohlenstoffatome auch Bestandteil eines aromatischen Ringsystems sein kann, A für einen n-wertigen organischen Rest steht und n eine ganze Zahl gleich oder größer 2 ist.

2. Verbindung nach Anspruch 1, worin A für einen gesättigten oder ungesättigten, linearen, verzweigten oder cyclischen aliphatischen Kohlenwasserstoffrest, welcher durch nicht benachbarte Sauerstoff-, Stickstoff- oder Schwefelatome unterbrochen sein kann, einen aromatischen Kohlenwasserstoffrest oder einen teilweise aliphatischen und aromatischen Kohlenwasserstoffrest steht.

3. Dispersion oder Lösung eines radikalischen Polymerisats, Polykondensats oder Polyaddukts, enthaltend eine Verbindung der Formel I.

4. Dispersion oder Lösung eines radikalischen Polymerisats, Polykondensats oder Polyaddukts, welches zu 0,001 bis 20 Gew.-% aus Aldehydgruppen - CHO - oder Ketogruppen - CO - besteht, enthaltend eine Verbindung der Formel I.

5. Dispersion oder Lösung nach Anspruch 3 oder 4, enthaltend als radikalisches Polymerisat Copolymerisate mit einer Glasübergangstemperatur zwischen -60 und +140°C, wobei das Copolymerisat aus
a) mindestens einem Comonomeren mit mindestens einer Aldehyd- oder Ketogruppe,
b) 20 bis 99,99 Gew.-% mindestens eines C₁- bis C₂₀-Alkyl(meth)acrylats, eines Vinylesters von 1 bis 20 C-Atome enthaltenden Carbonsäuren, eines Vinylaromaten mit bis zu 20 C-Atomen, eines ethylenisch ungesättigten Nitrils mit 3 bis 6 C-Atomen, eines Vinylhalogenids oder eines nichtaromatischen Kohlenwasserstoffs mit 4 bis 8 C-Atomen und mindestens 2 konjugierten Doppelbindungen, und
c) 0 bis 50 Gew.-% mindestens eines weiteren ethylenisch ungesättigten Monomeren besteht,
wobei der Gehalt des Monomeren a) so gewählt wird, daß das Copolymerisat zu 0,001 bis 20 Gew.-% aus Aldehyd -CHO- oder Ketogruppen -CO- besteht und die Monomeren a), b) und c) sich zu 100 Gew.-% addieren.

6. Verwendung einer Dispersion oder Lösung nach einem der Ansprüche 3 bis 5 als Beschichtungsmittel.

7. Verwendung einer Dispersion oder Lösung nach einem der Ansprüche 3 bis 5 als Klebstoff.

8. Beschichtete Substrate, erhältlich unter Verwendung einer Dispersion oder Lösung nach einem der Ansprüche 3 bis 5.

## Claims

1. A compound of the formula 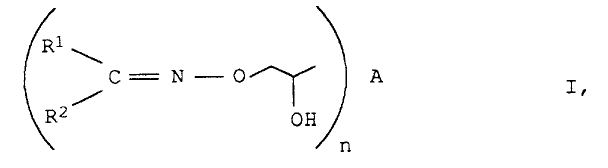 where R¹ and R², independently of one another, are each C₁-C₁₀-alkyl, C₁-C₁₀-alkoxy, C₅-C₁₀-cycloalkyl or C₅-C₁₀-aryl, each of which may furthermore contain from 1 to 3 nonadjacent nitrogen, oxygen or sulfur atoms as hetero atoms in the carbon chain or carbon ring and may be substituted by 1 to 3 C₁-C₄-alkyl or C₁-C₄-alkoxy groups, R¹ or R² may be hydrogen or R¹ and R² together form a bridge of 2 to 14 carbon atoms, where some of the carbon atoms may furthermore be part of an aromatic ring system, A is an n-valent organic radical and n is an integer equal to or greater than 2.

2. A compound as claimed in claim 1, wherein A is a saturated or unsaturated, linear, branched or cyclic aliphatic hydrocarbon radical which may be interrupted by nonadjacent oxygen, nitrogen or sulfur atoms, an aromatic hydrocarbon radical or a partly aliphatic and aromatic hydrocarbon radical.

3. A dispersion or solution of a free radical polymer, polycondensate or polyadduct, containing a compound of the formula I.

4. A dispersion or solution of a free radical polymer, polycondensate or polyadduct which comprises from 0.001 to 20% by weight of aldehyde groups -CHO- or keto groups -CO-, containing a compound of the formula I.

5. A dispersion or solution as claimed in claim 3 or 4, containing, as the free radical polymer, a copolymer having a glass transition temperature of from -60 to +140°C, the copolymer consisting of
a) at least one comonomer having at least one aldehyde or keto group,
b) from 20 to 99.99% by weight of at least one C₁-C₂₀-alkyl (meth)acrylate, one vinyl ester of a carboxylic acid of 1 to 20 carbon atoms, one vinylaromatic of up to 20 carbon atoms, one ethylenically unsaturated nitrile of 3 to 6 carbon atoms, one vinyl halide or one nonaromatic hydrocarbon having 4 to 8 carbon atoms and at least 2 conjugated double bonds, and
c) from 0 to 50% by weight of at least one further ethylenically unsaturated monomer,
the content of the monomer a) being chosen so that the copolymer comprises from 0.001 to 20% by weight of aldehyde groups -CHO- or keto groups -CO-, and the monomers a), b) and c) summing to 100% by weight.

6. Use of a dispersion or solution as claimed in any of claims 3 to 5 as a coating material.

7. Use of a dispersion or solution as claimed in any of claims 3 to 5 as an adhesive.

8. A coated substrate obtainable using a dispersion or solution as claimed in any of claims 3 to 5.

## Revendications

1. Composé de la formule : 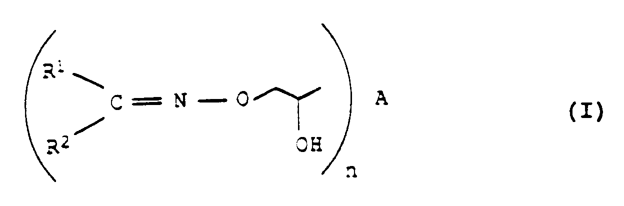 dans laquelle R¹ et R² représentent, chacun indépendamment l'un de l'autre, un radical alkyle en C₁ à C₁₀, alcoxy en C₁ à C₁₀, cycloalkyle en C₅ à C₁₀, ou aryle en C₅ à C₁₀, qui contiennent aussi de 1 à 3 atomes d'azote, d'oxygène, ou de soufre, non contigus, à titre d'hétéroatomes dans la chaîne carbonée ou dans le cycle carboné et qui peuvent être substitués de 1 à 3 radicaux alkyle ou alcoxy en C₁ à C₄, R¹ ou R² pouvant aussi représenter chacun un atome d'hydrogène, ou bien R¹ et R² forment ensemble un pont constitué de 2 à 14 atomes de carbone, où une partie des atomes de carbone peut aussi faire partie d'un système cyclique aromatique, A représente un radical organique n valant et n représente un nombre entier égal ou supérieur à 2.

2. Composé suivant la revendication 1, caractérisé en ce que A représente un reste d'hydrocarbure aliphatique, cyclique, ramifié, linéaire, insaturé, ou saturé, qui peut être interrompu par des atomes d'oxygène, d'azote, ou de soufre, non contigus, un reste d'hydrocarbure aromatique, ou un reste d'hydrocarbure partiellement aliphatique et aromatique.

3. Dispersion ou solution d'un polymère radicalaire, d'un polycondensat ou d'un polyadduit, contenant un composé' de la formule I.

4. Dispersion ou solution d'un polymère radicalaire, d'un polycondensat ou d'un polyadduit, qui se compose pour jusqu'à 0,001 à 20% en poids de radicaux aldéhyde -CHO- ou de radicaux céto -CO-, contenant un composé de la formule I.

5. Dispersion ou solution suivant la revendication 3 ou 4, contenant, à titre de polymère radicalaire, des copolymères d'une température de transition vitreuse comprise entre -60 et +140°C, où le copolymère se compose
a) d'au moins un comonomère avec au moins un radical aldéhyde ou céto,
b) de 20 à 99,99% en poids d'au moins un (méth)acrylate d'alkyle en C₁ à C₂₀, d'un ester vinylique d'acides carboxyliques contenant de 1 à 20 atomes de carbone, d'un composé vinyl-aromatique comportant jusqu'à 20 atomes de carbone, d'un nitrile éthyléniquement insaturé comportant de 3 à 6 atomes de carbone, d'un halogénure de vinyle ou d'un hydrocarbure non aromatique, comportant de 4 à 8 atomes de carbone et au moins 2 doubles liaisons conjuguées, et
c) de 0 à 50% en poids d'au moins un monomère éthyléniquement insaturé supplémentaire,
où la teneur du monomère a) est choisie en une manière telle que le copolymère se compose pour jusqu'à 0,001 à 20% en poids de radicaux aldéhyde -CHO- ou céto -CO- et les monomères a), b) et c) s'additionnent pour faire 100% en poids.

6. Utilisation d'une dispersion ou d'une solution suivant l'une quelconque des revendications 3 à 5 à titre d'agents de revêtement.

7. Utilisation d'une dispersion ou d'une solution suivant l'une quelconque des revendications 3 à 5 à titre de colle.

8. Supports revêtus, que l'on peut obtenir par l'emploi d'une dispersion ou d'une solution suivant l'une quelconque des revendications 3 à 5.
